# EUROPÄISCHE PATENTSCHRIFT

(11) **EP 3 194 436 B1**
(45) Veröffentlichungstag und Bekanntmachung des Hinweises auf die Patenterteilung: **19.08.2020**
(21) Anmeldenummer: 15808324.6
(22) Anmeldetag: 17.09.2015
(51) Int. Cl.: C07K 16/18, G01N 33/53

(54) **MONOKLONALER ANTIKÖRPER GEGEN HUMANES TAU-PROTEIN**
MONOCLONAL ANTIBODY AGAINST HUMAN TAU PROTEIN
ANTICORPS MONOCLONAUX DIRIGÉS CONTRE LA PROTÉINE TAU HUMAINE

(30) Priorität: 18.09.2014 DE 102014013571
(43) Veröffentlichungstag der Anmeldung: 26.07.2017
(73) Patentinhaber: Roboscreen GmbH, 04129 Leipzig (DE)
(72) Erfinder: LACHMANN, Ingolf, 04299 Leipzig (DE); HOLZER, Max, 04299 Leipzig (DE); LEWCZUK, Piotr, 91334 Hemhofen (DE); KORNHUBER, Johannes, 90491 Nürnberg (DE); PERRET-LIAUDET, Armand, 1255 Veyrier (CH); WANIEK, Katharina, 04177 Leipzig (DE)
(74) Vertreter: Rothe, Silke
(86) Internationale Anmeldenummer: PCT/DE2015/100394
(87) Internationale Veröffentlichungsnummer: WO 2016/041553

(56) Entgegenhaltungen:
- WO-A1-95/05466
- WO-A2-2014/100600
- US-A1- 2012 295 852
- L. I. BINDER ET AL: "The distribution of tau in the mammalian central nervous system", THE JOURNAL OF CELL BIOLOGY, Bd. 101, Nr. 4, 1. Oktober 1985 (1985-10-01), Seiten 1371-1378, XP055161258, ISSN: 0021-9525, DOI: 10.1083/jcb.101.4.1371
- N GHOSHAL: "Tau Conformational Changes Correspond to Impairments of Episodic Memory in Mild Cognitive Impairment and Alzheimer's Disease", EXPERIMENTAL NEUROLOGY, Bd. 177, Nr. 2, 1. Oktober 2002 (2002-10-01), Seiten 475-493, XP055089574, ISSN: 0014-4886, DOI: 10.1006/exnr.2002.8014
- PUSHKO P ET AL: "IDENTIFICATION OF HEPATITIS B VIRUS CORE PROTEIN REGIONS EXPOSED OR INTERNALIZED AT THE SURFACE OF HBCAG PARTICLES BY SCANNING WITH MONOCLONAL ANTIBODIES", VIROLOGY, ELSEVIER, AMSTERDAM, NL, vol. 202, no. 2, 1 August 1994 (1994-08-01), pages 912-920, XP002035985, ISSN: 0042-6822, DOI: 10.1006/VIRO.1994.1413
- ANONYMOUS: "Anti-Tau (T175) Antibody", DATASHEET, 29 April 2014 (2014-04-29),
- JOERY GOOSSENS ET AL: "No added diagnostic value of non-phosphorylated tau fraction (p-taurel) in CSF as a biomarker for differential dementia diagnosis", ALZHEIMER'S RESEARCH & THERAPY, vol. 9, no. 1, 14 July 2017 (2017-07-14), pages 1-7, DOI: 10.1186/s13195-017-0275-5
- ANONYMOUS: "Mouse Anti-TAU-1 monoclonal antibody / MAB3420", DATASHEET, January 2014 (2014-01),
- EBRU ERCAN ET AL: "A validated antibody panel for the characterization of tau post-translational modifications", MOLECULAR NEURODEGENERATION, vol. 12, no. 1, 21 November 2017 (2017-11-21), DOI: 10.1186/s13024-017-0229-1
- ERMANN NATALIA ET AL: "CSF nonphosphorylated Tau as a biomarker for the discrimination of AD from CJD.", ANNALS OF CLINICAL AND TRANSLATIONAL NEUROLOGY JUL 2018, vol. 5, no. 7, July 2018 (2018-07), pages 883-887, ISSN: 2328-9503
- MOLINUEVO JOSÉ LUIS ET AL: "Current state of Alzheimer's fluid biomarkers", ACTA NEUROPATHOLOGICA, SPRINGER VERLAG, BERLIN, DE, vol. 136, no. 6, 28 November 2018 (2018-11-28), pages 821-853, ISSN: 0001-6322, DOI: 10.1007/S00401-018-1932-X [retrieved on 2018-11-28]
- CALDERÓN-GARCIDUEÑAS LILIAN ET AL: "Non-Phosphorylated Tau in Cerebrospinal Fluid is a Marker of Alzheimer's Disease Continuum in Young Urbanites Exposed to Air Pollution.", JOURNAL OF ALZHEIMER'S DISEASE : JAD 2018, vol. 66, no. 4, 2018, pages 1437-1451, ISSN: 1875-8908
- ILLENBERGER S ET AL: "The endogenous and cell cycle-dependent phosphorylation of tau protein in living cells: implications for Alzheimer's disease", MOLECULAR BIOLOGY OF THE CELL, AMERICAN SOCIETY FOR CELL BIOLOGY, US, vol. 9, 1 June 1998 (1998-06-01), pages 1495-1512, ISSN: 1059-1524
- ROSENBERG AMY S: "Effects of protein aggregates: An immunologic perspective", THE AAPS JOURNAL, SPRINGER NEW YORK, NEW YORK, vol. 8, no. 3, 1 September 2006 (2006-09-01), pages E501-E507, DOI: 10.1208/AAPSJ080359 [retrieved on 2006-09-01]
- HESTER A DOYLE ET AL: "Autoantigenesis: the evolution of protein modifications in autoimmune disease", CURRENT OPINION IN IMMUNOLOGY., vol. 24, no. 1, 29 December 2011 (2011-12-29), pages 112-118, GB ISSN: 0952-7915, DOI: 10.1016/j.coi.2011.12.003

## Beschreibung

Die vorliegende Erfindung bezieht sich auf einen monoklonalen Antikörper gegen humanes TAU-Protein sowie Verwendungen des monoklonalen Antikörpers.

Monoklonale Antikörper sind immunologisch aktive Proteine, die von einer Zelllinie produziert werden, die auf einen einzigen B-Lymphozyten zurückgeht. Monoklonale Antikörper richten sich gegen ein einzelnes Epitop. In der Diagnostik und Forschung spielen monoklonale Antikörper folglich eine große Rolle, da sie mit hoher Spezifität eine Anzahl von Molekülen binden können, wobei sich die Bindung mit unterschiedlichen Techniken nachweisen lässt.

Verfahren zur Herstellung von monoklonalen Antikörpern gehen auf Arbeiten von Köhler & Milstein [1] zurück und sind in den vergangenen Jahren und Jahrzehnten breit erforscht und ausgebaut worden. Monoklonale Antikörper sind als Detektoren für Epitope auf Zelloberflächen unverzichtbar. In der Diagnostik von Krankheiten an Tier und Mensch werden sie vielfältig eingesetzt, als Bestandteile von Therapeutika ebenso.

Die Alzheimer'sche Erkrankung (*morbus Alzheimer*) ist charakterisiert durch das Auftreten von zwei Formen von Proteinaggregaten im Gehirn von betroffenen Patienten. Extrazelluläre Amyloid-Plaques und intraneuronale Verklumpungen des TAU-Proteins im Isokortex sind die pathologischen Marker zur *post mortem* Diagnose der Alzheimer'schen Erkrankung. Beide Proteinbestandteile dieser Ablagerungen, das Beta-Amyloid-Peptid und das TAU-Protein, können als potentielle Biomarker zur Diagnose der Erkrankung dienen. Eine Abnahme des Gehaltes des Beta-Amyloid-Peptids und eine Zunahme an TAU-Protein und dessen phosphorylierter Form an Aminosäure T181 im Liquor sind Kennzeichen einer fortgeschrittenen Alzheimer'schen Erkrankung (Lewczuk & Kornhuber [2]) und können eine klinische Diagnose stützen.

Das TAU-Protein existiert im menschlichen Gehirn in sechs Isoformen, die entwicklungsabhängig exprimiert werden (Kosik et al. [3]; Goedert et al. [4]). Die sogenannten Splicing-Isoformen entstammen einem einzigen TAU-Gen, das insgesamt 14 Exons enthält. Der Gen-Locus befindet sich auf dem langen Arm des Chromosoms 17 (Neve et al. [5]). Die molekulare Heterogenität der TAU-Expression im zentralen Nervensystem entsteht durch alternatives Spleißen der Exons 2, 3 und 10 (Goedert et al. [6]). Dabei werden zwei Sequenzen von je 29 Aminosäuren in der N-terminalen Hälfte bzw. eine zusätzliche Mikrotubulibindung in der C-terminalen Region eingefügt.

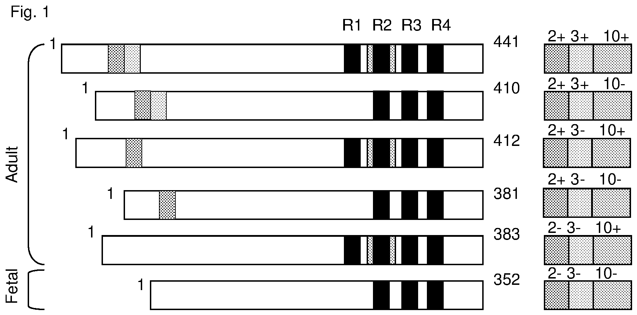

Figur 1 zeigt hierzu eine schematische Darstellung der TAU-Isoformen. Die Bezeichnung der einzelnen Isoformen erfolgt nach Goedert [7]. Die alternativ benutzten Exons 2, 3 und 10 sind hervorgehoben. Die Lokalisation der Mikrotubuli-Bindungsrepeats ist schwarz dargestellt.

Aufgrund seiner Aminosäure-Zusammensetzung ist das TAU-Protein hydrophil, gut löslich und liegt in einem nativ ungefalteten Zustand (ohne Sekundärstruktur) vor. Die Auslöser, welche die Aggregation des TAU-Proteins *in vivo* unter pathologischen Bedingungen verursachen, sind unbekannt. Es wird angenommen, dass es durch Interaktionen mit anderen Proteinen, die Konformationsänderungen oder posttranslationale Veränderungen des TAU-Proteins verursachen können, und/oder durch Mutationen (im Falle von FTDP-17) im TAU-Gen zur Aggregation des TAU-Proteins kommen kann. *In vitro* konnte gezeigt werden, dass die meisten exonischen Mutationen im TAU-Gen die Fähigkeit des TAU-Proteins, an Mikrotubuli zu binden, beeinträchtigen, seine Aggregationstendenz erhöhen oder zu einem veränderten Verhältnis der TAU-Isoformen führen (Hutton et al. [8]; Bugiani et al. [9]). Mit zunehmendem Phosphorylierungsgrad und bei Hyperphosphorylierung des TAU-Proteins sinkt dessen Affinität, Mikrotubuli zu binden und Mikrotubuli-Polymerisation zu vermitteln (Eidenmüller et al. [10]). Durch Mutationen und posttranslationale Hyperphosphorylierung kann es also zur Akkumulation von hyperphosphoryliertem TAU-Protein im Zytoplasma von Neuronen kommen. Diese Akkumulation von hyperphosphoryliertem TAU-Protein im Soma wird als erster detektierbarer Schritt der TAU-Aggregation akzeptiert (Bancher et al. [11]). Es wird angenommen, dass die akkumulierten, hyperphosphorylierten TAU-Monomere in einen entropiegetriebenen Kernbildungs-Kondensationsmechanismus über Zwischenstufen zu TAU-Filamenten aggregieren (Friedhoff et al. [12]; Chirita & Kuret [13]). Dabei kommt es im Bereich der Mikrotubuli-Bindungsregion zu einer Konformationsänderung vom sog. "random coil" hin zur beta-Faltblatt-Sekundärstruktur (Berriman et al. [14]). Nach der Konformationsänderung kommt es in dem zeitbestimmenden Schritt der TAU-Aggregation zur Bildung eines Dimers, die vermutlich auf der hydrophoben Wechselwirkung der beta-Faltblatt-Strukturen beider Moleküle beruht. Dabei lagern sich die beiden TAU-Moleküle überkreuzt, d.h. um 90° versetzt über ihre beta-Faltblatt-Strukturen aneinander an (cross-β-Zustand). Über die Ausbildung von intermolekularen Disulfid- und Wasserstoffbrücken kann das Dimer stabilisiert werden. Durch Zusammenlagerung von 4 bis 7 Dimeren entsteht der sogenannte Kern bzw. der Kondensationskeim, an den sich weitere Dimere anlagern.

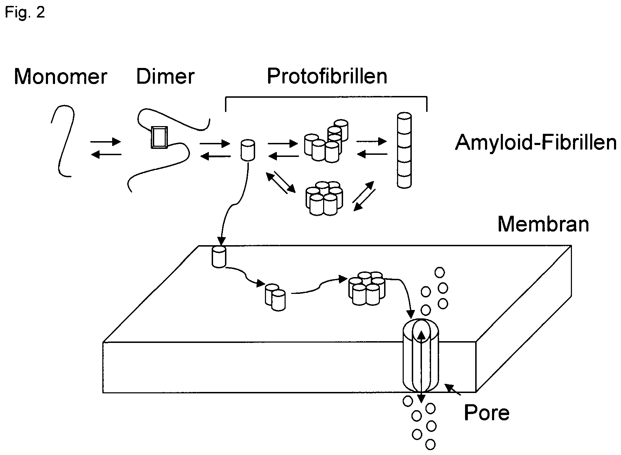

Figur 2 zeigt hierzu die Aggregation amyloidogener Proteine und Ausbildung von Poren in der Zellmembran. Die Aggregation amyloidogener Proteine wird vermutlich durch Konformationsänderungen im Protein-Monomer hin zu beta-Faltblatt-Strukturen eingeleitet. Während des zeitbestimmenden Schritts der Protein-Aggregation kommt es durch hydrophobe Wechselwirkungen zwischen den ausgebildeten beta-Faltblatt-Strukturen zweier Monomere zur Ausbildung von Dimeren. Es wird vermutet, dass Oligomere amyloidogener Proteine in der Zellmembran Poren ausbilden können, die zu einer Störung der Membranintegrität führen (modifiziert nach Lashuel [15]).

Zunächst entstehen dabei kleine, granuläre TAU-Oligomere, die aus ca. 40 Monomeren bestehen (Maeda et al. [16]). Durch weitere Anlagerung von Dimeren entstehen aus den granulären TAU-Oligomeren dann größere, protofibrilläre Oligomere. Aus den protofibrillären Oligomeren bilden sich dann durch fortgesetzte Anlagerung von Dimeren die TAU-Filamente. Mehrere TAU-Filamente bilden schließlich die für Tauopathien typischen Neurofibrillenbündel (Caughey & Lansbury [17]) .

Die Relevanz von TAU-Proteinen ist im Stand der Technik prinzipiell bekannt. So beschreibt US 6,232,437 B1 verschiedene TAU-Peptid-Epitope in Verbindung mit dem monoklonalen Antikörper AT120, der sowohl mit normalen als auch mit abnormal phosphoryliertem TAU reagiert und eine Bindungsstelle der Aminosäure-Sequenz P218 bis L224 des TAU-Proteins erkennt. Ferner beschreibt US 6,238,892 B1 einen monoklonalen Antikörper AT8, der einen immunologischen Komplex mit einem phosphorylierten Epitop eines Antigens bildet, das zu den humanen abnorm phosphorylierten TAU-Proteinen gehört.

Die aus dem Stand der Technik bekannte sehr gute Charakterisierung von Antikörpern, welche die Hyper-Phosphorylierung des TAU-Proteins detektieren, beruht auf der Tatsache, dass diese Phosphorylierungen des TAU-Proteins einen abnormalen Zustand darstellen und damit Antikörper, die dieses phosphorylierte TAU erkennen, besonders gut geeignet sind, pathologische Zustände zu erfassen.

Die aus der Literatur bekannten Techniken beruhen darauf, dass abnormal phosphoryliertes TAU pathologisch relevant ist und dass Antikörper für einen Nachweis herzustellen und zu verwenden sind, die dieses abnormal phosphorylierte TAU und insbesondere genau die Phosphorylierung erkennen. Ferner ist deren Bindungsstelle am abnormal phosphorylierten TAU zerstört, wenn eine Dephosphorylierung vorliegt.

In WO 2014/100600 A2 werden neue humane Tau-spezifische Antikörper sowie Fragmente, Derivate und Varianten davon und die damit verbundenen Methoden zur Verfügung gestellt, sowie Assays, Kits und feste Träger, die mit Tau-spezifischen Antikörpern in Verbindung stehen. Der Antikörper, die Immunglobulinkette(n) sowie die Bindungsfragmente, Derivate und Varianten davon können in pharmazeutischen und diagnostischen Zusammensetzungen für die gezielte Immuntherapie bzw. Diagnose von Tau verwendet werden. Konkret beschreibt WO 2014/100600 A2 die Herstellung von humanen Antikörpern gegen humanes TAU unter der Verwendung von synthetischen Peptidstrukturen, die aufgelistet und in Anspruch 1 benannt sind. Diese benannten Peptide sind in den Figuren 7A bis 7O dargestellt. In Anspruch 2 wird unter Punkt iii) die Bindung an pathologisches TAU, in Punkt iv) die Bindung an nichtphysiologischen Formen von TAU und in Punkt x) die Aminosäuresequenzen des TAU definiert, wobei explizit die Bereiche 175-177 (TPP), 181-183 (TPP) nicht enthalten sind und 231-233 (TPP) nur mit T (231) beschrieben wird.

WO 1995/005466 A1 geht auf Transgene Zell- und Tiermodelle für die Alzheimer-Krankheit ein. Zellen der Tiere und die Zellmodelle selbst umfassen ein rekombinantes DNA-Konstrukt, das eine Kontrollsequenz und unter der Kontrolle der Kontrollsequenz eine DNA-Sequenz umfasst, die für eine Kinase kodiert, die direkt oder indirekt in der Lage ist, die Phosphorylierung des Mikrotubuli bildendes Proteins Tau zu modulieren. Die transgenen Zellen und Tiere können für die Prüfung potenzieller therapeutischer Wirkstoffe für die Alzheimer-Krankheit verwendet werden. WO 1995/005466 A1 offenbart ein Zellkulturmodell, das TAU produziert, welches phosphoryliert und für die Testung von therapeutischen Agenzien verwendet werden kann. Es kann daher weder ein direkter noch ein indirekter Bezug zur vorliegenden Erfindung erkannt werden.

L. I. Binder et al. haben in "The distribution of tau in the mammalian central nervous system." die biochemische und immunzytochemische Lokalisierung des heterogenen Mikrotubuli assoziierten Proteins Tau mit Hilfe eines monoklonalen Antikörpers beschrieben, der an alle Tau-Polypeptide sowohl im Rinder- als auch im Rattenhirn bindet. Unter Verwendung von Immunoblot-Assays und kompetitiven enzymgebundenen Immunosorbent-Assays haben sie gezeigt, dass Tau in Extrakten und Mikrotubuli der weißen Substanz von Rindern häufiger vorkommt als in Extrakten und Mikrotubuli aus einer angereicherten Region der grauen Substanz des Gehirns. Ihre Beobachtungen deuteten darauf hin, dass Tau helfen könnte, eine Subpopulation von Mikrotubuli zu definieren, die auf Axone beschränkt ist. Darüber hinaus sollte sich der beschriebene monoklonale Antikörper als sehr nützlich für Forscher erweisen, die die axonale Keimung und das Wachstum untersuchen, da er ein exklusiver axonaler Marker ist. Konkret wurde ein Mikrotubuli bindendes Protein (TAU) aus Rindern bestimmt, das zur Immunisierung von Mäusen verwendet wurde und daraus einen Antikörper erzeugte, der TAU an den Aminosäuren 192-204 bindet (vgl. Szendrei GI et al. 1993, J. Neurosci Res 1, 34 (2), 243-249).

Aus dem Stand der Technik und den zitierten wissenschaftlichen Aufsätzen ist folglich bislang kein Antikörper bekannt, mit dem es möglich ist und der für diesen Zweck erfunden wurde, eine Fraktion nachzuweisen, die an für pathologische Prozesse bisher bekannten Phosphorylierungsstellen nicht phosphoryliert ist.

Ausgehend von den vorstehend genannten Nachteilen des Standes der Technik liegt der vorliegenden Erfindung die Aufgabe zugrunde, einen monoklonalen Antikörper zur Verfügung zu stellen, der spezifisch das TAU-Protein erkennt, das an den Aminosäuren T175 und/oder T181 und/oder T231 nicht phosphoryliert ist.

Ferner ist es Ziel der vorliegenden Erfindung, Verwendungen des monoklonalen Antikörpers zu entwicklen.

Die vorstehend genannte Aufgabe wird in einem ersten Aspekt der vorliegenden Erfindung durch den monoklonalen Antikörper 1G2, produziert durch die Hybridomzelllinie H-1G2, gekennzeichnet durch DSM ACC3248, gelöst. Dieser monoklonaler Antikörper 1G2 zeigt eine hohe Spezifität für ein humanes TAU-Protein, das an den Phosphorylierungsstellen T175 und T181 und T231 nicht phosphoryliert ist. Der erfindungsgemäße monoklonale Antikörper 1G2 bindet im Gegensatz zu aus dem Stand der Technik bekannten Antikörpern nicht an abnormal phosphoryliertes TAU-Protein, sondern erkennt nicht-abnormales, d.h. natürliches TAU-Protein. Die Bindung und damit die Erkennung ist besonders stark, wenn für den erfindungsgemäßen Antikörper 1G2 mindestens zwei oder alle drei Positionen T175 und T181 und T231 zur Verfügung stehen.

Zudem bindet der erfindungsgemäße monoklonale Antikörper 1G2 unabhängig von der Wirkungsweise von Phosphatasen, wie sie in der Literatur teilweise beschrieben werden. Darüber hinaus bindet der Antikörper alle bekannten Isoformen des TAU-Proteins.

In einem zweiten Aspekt wird die vorstehend genannte Aufgabe durch die Hybridomzelllinie H-1G2 gelöst, die durch die Hinterlegung mit der Nummer DSM ACC3248 vom 15. Juli 2015 gekennzeichnet ist. Diese Hybridomzelllinie produziert und sezerniert den erfindungsgemäßen monoklonalen Antikörper 1G2 vom Isotyp IgG2a.

Das Epitop TPP, umfassend die Aminosäuren Threonin, Prolin, Prolin, ist spezifisch als Aminosäuresequenz für Proteine des Menschen, insbesondere für das TAU-Protein. Die Aminosäuren Threonin, Prolin, Prolin sind im N-Terminus flankiert durch eine andere, basische Aminosäure, die entweder R (Arginin) oder K (Lysin) ist. Diese flankierenden Aminosäuren sind für die Bindung an den erfindungsgemäßen monoklonalen Antikörper 1G2 notwendig, da im TAU-Protein zweimal die Sequenz KTTP und einmal die Sequenz RTTP vorhanden ist, welche von diesem Antikörper gebunden werden.

Die flankierende Wirkung dieser basischen Aminosäuren auf die Bindungsfähigkeit des Antikörpers an der Sequenz TPP weist darauf hin, dass geringste Änderungen in der Umgebung der Bindungsstelle des Antikörpers, zum Beispiel durch weitere Phosphorylierungen nicht an den Bindungsstellen des Antikörpers 1G2 sondern an anderen Aminosäuren des TAU-Proteins, von denen zum Beispiel in der Umgebung der Bindungsstellen u. a. die Aminosäuren 184, 195, 198, 199, 202, 205, 208, 210, 212, 214, 217, 235, 237, 238 bekannt sind, die Bindung des Antikörpers 1G2 unterbinden könnten.

Ein Aspekt außerhalb der vorliegenden Erfindung betrifft ein Verfahren zur Herstellung des erfindungsgemäßen monoklonalen Antikörpers gegen eine Fraktion des TAU-Proteins, die an den Positionen T175 und T181 und T231 nicht phosphoryliert ist, umfassend die Schritte
a) Immunisierung von Säugern oder in *in-vitro-*Zellkulturen mit rekombinant hergestelltem und in vitro aggregiertem alle Aminosäuren umfassenden TAU-Protein, das mittels Kinasen *in vitro* phosphoryliert wurde, zur Induktion einer spezifischen Immunantwort gegen das TAU-Protein in einem Adjuvans,
b) Entnahme von B-Lymphozyten aus den Säugern oder *in vitro* Zellkulturen,
c) Fusion der B-Lymphozyten mit einer murinen Myelomzelllinie, die für eine nachfolgende Selektion von Fusionszellen geeignet ist, unter Erhalt von Fusionsprodukten,
d) HAT-Selektion der Fusionsprodukte in Zellkulturgefäßen bei spezifischen Kulturbedingungen unter Erhalt von Hybridomen,
e) Auswahl von Hybridomen, die unter den Selektionsbedingungen wachsen und Immunglobulin produzieren, aufgrund ihrer Reaktivität gegenüber phosphorylierten und nicht-phosphorylierten TAU-Peptiden sowie dem TAU-Protein,
f) Vereinzeln der Hybridome auf Einzelzellebene zur Sicherstellung der Monoklonalität der Hybridom-Kultur,
g) Kultivierung der vereinzelten Hybridome als Hybridomzelllinie *in vitro* oder *in vivo* unter Erhalt des erfindungsgemäßen monoklonalen Antikörpers, der das Epitop TPP auf einem TAU-Protein erkennt.

Die Fraktion des TAU-Proteins kann dabei sowohl das komplette TAU-Protein mit 441 Aminosäuren (im Folgenden auch als "TAU441" bezeichnet), dessen Splicing-Formen wie auch degradiertes TAU-Protein in biologischen Proben sowie aggregiertes TAU-Protein sein, das an den Positionen T175 und T181 und T231 nicht phosphoryliert ist.

Die Immunisierung von Säugern in Schritt a) bezieht sich vorzugsweise auf Mäuse (*mus musculus*), die zu diesem Zweck gezüchtet und gehalten werden.

Unter der in Schritt a) genannten spezifischen Immunantwort wird insbesondere eine auf Immunglobulin G basierende Immunantwort gegen das TAU-Protein verstanden, was bislang aufgrund der Homologie von humanem und murinem TAU-Protein eine erhöhte Schwierigkeit für die Induktion einer solchen Immunantwort darstellte. Diese Schwierigkeit konnte von den gegenwärtigen Erfindernüberwunden werden, was dadurch erreicht wurde, dass mit phosphorylierten Antigenen immunisiert wurde und durch diese Veränderung die natürlich ausgebildete Toleranz gegen körpereigene Antigene - wobei hier die hohe Homologie als Möglichkeit einer bestehenden Toleranz anzusehen ist - umgangen wird und ebenso mit *in vitro* erzeugten Aggregaten des TAU eine durch Toleranz schwer induzierbare Immunantwort erzeugt werden kann.

Das in Schritt a) eingesetzte Adjuvans unterstützt die Entwicklung einer T-Helferzellantwort zur Unterstützung der Erzeugung spezifischer B-Zellen. Es ist zum Beispiel ein komplettes Freund'sches Adjuvans.

Die murine Myelomzellliniein Schritt c), die für eine nachfolgende Selektion von Fusionszellen geeignet ist, stellt vorzugsweise eine B-Zelllinie dar, die selbst keine oder nur unvollständige Immunglobuline bildet und einen wirksamen Defekt für ein HAT-basierendes Selektionsprinzip besitzt. Dies ist beispielsweise die Linie X63Ag8.653. Vorzugsweise wird in diesem Schritt Polyethylenglycol 1.500 (mittlere relative Molekülmasse 1.500) verwendet.

Unter den erhaltenen Fusionsprodukten werden insbesondere Zellen verstanden, die durch Verschmelzung von Zellkörper, Zellkern und genetischem Material aus den eingesetzten Myelom-Zelllinien und den isolierten B-Zellen entstanden sind und weiterhin die Eigenschaften des unbegrenzten Wachstums der Myelom-Zellen und des kompletten Mechanismus der Purin-Synthese sowie der Produktion von Immunglobulinen der B-Zellen zeigen.

Bei der HAT-Selektion in Schritt d) werden die spezifischen Kulturbedingungen durch die Verwendung eines Mediums erreicht, welches das differenzierte Wachstum von Mauszellen befördert, und in einer Atmosphäre, die mit Kohlendioxid angereichert eine Temperatur von 30 °C bis 40 °C hat. Die damit erhaltenen Hybridome sind vorzugsweise Fusionszellen aus Myelom-Zellen und B-Zellen, die unter dem Selektionsdruck von HAT selektiv wachsen können und die noch nicht durch eine spezifische Reaktivität bzw. die Produktion von Antikörpern charakterisiert sind.

Die Auswahl der Hybridome in Schritt e) erfolgt insbesondere aufgrund ihrer Reaktivität gegenüber den TAU-Peptiden, besonders deren nicht phosphorylierten Formen sowie TAU441.

Das Konzept der vorliegenden Erfindung wird dadurch verwirklicht, dass der erfindungsgemäße monoklonale Antikörper 1G2 durch das vorstehend dargestellte Verfahren mittels der erfindungsgemäßen Hybridomzelllinie hergestellt wird und für dessen Bindung an das TAU-Protein sein erfindungsgemäßes Epitop notwendig ist.

Als Vorteil der vorliegenden Erfindung stellt sich heraus, dass der erfindungsgemäße Antikörper 1G2 (und das darauf basierende immunologische Nachweissystem) der erste dieser Art ist, der gegenüber den bisher verwendeten Messungen von gesamtem TAU und phosphoryliertem Tau (P-TAU), das vor allem an Position 181 phosphoryliert nachgewiesen wird (Blennow et al. [18]), nunmehr eine Fraktion nachweist, die an den bekanntesten Phosphorylierungsstellen nicht phosphoryliert ist, und der damit neue Möglichkeiten der Erweiterung der bisher existierenden diagnostischen Möglichkeiten eröffnet.

Die vorliegende Erfindung beruht folglich darauf, einen bisher nicht beschriebenen und unabhängig vom bekannten Stand der Technik entwickelten Ansatz zu verfolgen, das TAU-Protein nachzuweisen, das nicht dem Phosphorylierungsgeschehen zur Bildung von abnormal phosphoryliertem TAU unterliegt, das pathologisch relevant ist.

In einem weiteren Aspekt bezieht sich die vorliegende Erfindung auf die Verwendung des erfindungsgemäßen monoklonalen Antikörpers 1G2 zur Erkennung in vitro einer Fraktion des TAU-Proteins, die an den Positionen T175 und T181 und T231 nicht phosphoryliert ist. Diese Fraktion kann sowohl das komplette TAU-Protein mit 441 Aminosäuren, dessen Splicing-Formen wie auch degradiertes TAU-Protein in biologischen Proben sowie aggregiertes TAU-Protein sein, das an den Positionen T175 und T181 und T231 nicht phosphoryliert ist. Damit ist der Antikörper geeignet, alle bekannten TAU-Formen in biologischen Proben zu erfassen, insbesondere unabhängig davon, ob diese im Prozess der Phosphorylierung/De-Phosphorylierung im Vorhandensein einer Phosphatgruppe an den benannten Phosphorylierungsstellen variieren.

In einer bevorzugten Weiterbildung dieser Verwendung wird der erfindungsgemäße monoklonale Antikörper 1G2 zum Nachweis der Fraktion des TAU-Proteins in vitro in biologischen Proben von Menschen eingesetzt. Diese Verwendung des monoklonalen Antikörpers 1G2 als sog. Fang-Antikörper bietet für seine spezifische Bindung ein hohes Maß an Sicherheit, da - wie bereits dargestellt - die Bindungsfähigkeit für Phosphor in den benannten Positionen und möglicherweise auch den benachbarten Positionen eine wesentliche Inhibierung dieser Bindung darstellt. Zudem verfügt der monoklonale Antikörper 1G2 durch die Möglichkeit einer gleichzeitigen Bindung von zwei Epitopen an einem TAU-Protein auch über eine besonders hohe Bindungsstärke, d.h. im konkreten Fall eine besonders hohe Avidität.

Ein anderer Aspekt außerhalb der vorliegenden Erfindung betrifft die Verwendung des erfindungsgemäßen monoklonalen Antikörpers 1G2, seiner humanisierten Form oder eines Moleküls mit den spezifischen Bindungseigenschaften des erfindungsgemäßen Antikörpers 1G2 an die Aminosäuresequenz TPP zur Herstellung eines Therapeutikums für mit dem TAU-Protein assoziierte Krankheiten beim Menschen, wobei in dem Therapeutikum der erfindungsgemäße monoklonale Antikörper 1G2, seine humanisierte Form oder ein Molekül mit den spezifischen Bindungseigenschaften des erfindungsgemäßen Antikörpers 1G2 an die Aminosäuresequenz TPP als wichtigster Bestandteil verantwortlich für die therapeutische Wirkung ist. Insbesondere kann der erfindungsgemäße monoklonale Antikörper 1G2, seine humanisierte Form oder ein Molekül mit den spezifischen Bindungseigenschaften des erfindungsgemäßen Antikörpers 1G2 an die Aminosäuresequenz TPP an das TAU-Protein als wichtigsten Bestandteil jener intrazellulären Aggregate binden, die sich im Gehirn von Patienten mit der Alzheimer'schen Erkrankung bilden. Bei Einsatz dieses Therapeutikums kann der erfindungsgemäße monoklonale Antikörper 1G2, seine humanisierte Form oder ein Molekül mit den spezifischen Bindungseigenschaften des erfindungsgemäßen Antikörpers 1G2 an die Aminosäuresequenz TPP in der Lage sein, Phosphorylierungsstellen für die ablaufenden Phosphorylierungsprozesse zu blockieren, insbesondere an Positionen, die Prozesse der Hyperphosphorylierung einleiten bzw. besonders die physiologische Bindung des TAU-Proteins an Mikrotubuli unterbinden (Braithwaite et al. [19]).

Unter "humanisierte Form" des erfindungsgemäßen Antikörpers 1G2 wird verstanden, dass Teile des oder das komplette Maus-Immunglobulin, aus dem der Antikörper besteht, mittels verfügbarer gentechnischer Methoden in humanes Immunglobulin umgewandelt wird (Cambridge University Press [20]).

Die vorstehend beschriebene Aufgabe wird in einem weiteren Aspekt der vorliegenden Erfindung durch ein immunochemisches Nachweissystem gelöst, umfassend
i) den erfindungsgemäßen monoklonalen Antikörper 1G2 als Fang-Antikörper,
ii) einen zweiten monoklonalen Antikörper gegen ein TAU-Protein als Nachweis-Antikörper,
iii) zumindest eine Kontrolle,
iv) zumindest eine Färbe- und/oder Nachweislösung,
v) zumindest eine Stopplösung,
vi) zumindest einen Probenpuffer,
vii) zumindest einen Waschpuffer und
viii) Mittel zur Probenvorbereitung.

Unter dem Begriff "immunochemisches Nachweissystem" ist eine Zusammenstellung von Bestandteilen zu verstehen (auch "Testsatz" oder "Testkit"), die zum Nachweis eines spezifischen Ziels, hier einer Fraktion des TAU-Proteins, die an wichtigen Positionen der Aminosäuresequenz nicht phosphoryliert ist, verwendet zu werden.

Der erfindungsgemäße monoklonale Antikörper 1G2 kann in diesem immunochemischen Nachweissystem zum Beispiel auf einer Mikrotiterplatte beschichtet sein. "Fang-Antikörper" bedeutet in diesem Zusammenhang, dass der Antikörper immobilisiert ist und das Antigen bindet, d.h. "fängt".

Der zweite monoklonale Antikörper ii) kann ein geeigneter Antikörper gegen das humane TAU-Protein oder ein TAU-Protein einer anderen Spezie sowie andere geeignete Konjugationen eines Nachweis-Antikörpers sein. Bevorzugt ist zweite monoklonale Antikörper der monoklonale Antikörper 7E5 (monoklonaler Antikörper IgG3[kappa], spezifisch gegen humanes TAU, konjugiert an Meerettich-Peroxidase). "Nachweis-Antikörper" bedeutet in diesem Zusammenhang, dass das durch den Fang-Antikörper gefangenen Antigen mit diesem Antikörper detektiert werden kann.

Die zumindest eine Kontrolle iii) dient dazu, die Funktionalität des immunochemischen Nachweissystems zu zeigen. Sie kann sich aus negativen Kontrollen (z.B. Probenpuffer) und positiven Kontrollen (z.B. rekombinantes TAU-Protein oder synthetische Peptide, die die Epitope der Antikörper enthalten und miteinander oder über einen Träger miteinander verbunden sind, in Probenpuffer portioniert und lyophilisiert) zusammengesetzt sein.

Die zumindest eine Färbe- und/oder Nachweislösung iv) wird in Abhängigkeit vom gewählten Nachweis eingesetzt, z.B. Meerettichperoxidase gekoppelt an den Nachweisantikörper. Erfindungsgemäß ist als Färbe-Lösung ein Kaliumcitratpuffer pH 3,0 bis pH 6,0 mit TMB-Lösung (10 mM - 100 mM) und Peroxid-Lösung (50 mM - 500 mM) bevorzugt.

Als zumindest eine Stopplösung v) wird in der vorliegenden Erfindung vorzugsweise 0,01 M bis 0,5 M Schwefelsäure verwendet.

Der zumindest eine Probenpuffer vi) ist bevorzugt 10 mM bis 200 mM phosphatgepufferte Kochsalzlösung pH 7 bis pH 8 mit 1 % bis 5 % BSA und 0,01 % bis 0,1% Tween 20.

Als der zumindest eine Waschpuffer vii) wird erfindungsgemäß 0,1 M bis 1 M Tris pH 7 bis pH 8, 0,5 M bis 5 M NaCl, 0,01 % bis 0,1 % Triton X100 eingesetzt.

Unter dem Mittel zur Probenvorbereitung viii) wird zum Beispiel die Vorbehandlung mit Detergenzien oder mittels physikalischer Faktoren wie Hitze oder Ultraschall verstanden

Ein weiterer Aspekt zur Lösung der vorstehend gennnten Aufgabe betrifft die Verwendung des erfindungsgemäßen immunochemischen Nachweissystems in der Humandiagnostik in vitro zur Untersuchung von Blutplasma, Blutserum, Liquor oder anderen Körperflüssigkeiten auf eine Fraktion des TAU-Proteins, die an den Positionen T175 und T181 und T231 nicht phosphoryliert ist. Diese Fraktion des TAU-Proteins ist sowohl das komplette TAU-Protein mit 441 Aminosäuren, dessen Splicing-Formen wie auch degradiertes TAU-Protein in biologischen Proben sowie aggregiertes TAU-Protein, dass an den Positionen T175 und T181 und T231 nicht phosphoryliert ist. Damit werden alle bekannten TAU-Formen in biologischen Proben erfasst, insbesondere unabhängig davon, ob diese im Prozess der Phosphorylierung/Dephosphorylierung im Vorhandensein einer Phosphatgruppe an den benannten Phosphorylierungsstellen variieren.

Ein anderer Aspekt außerhalb der vorliegenden Erfindung bezieht sich auf die Verwendung des erfindungsgemäßen immunochemischen Nachweissystems in der neurochemischen Demenzdiagnostik beim Menschen. Hier wird insbesondere auf die Diagnose von Patienten mit Alzheimer'scher Erkrankung abgezielt, wobei eine Abgrenzung dieser Patienten von anderen Patienten ohne neurodegenerativen Erkrankungen mittels eines definierten Grenzwerts basierend auf den ermittelten Daten einer klinischen Prüfung vorgenommen wird. Kurz dargestellt wurden die Messungen für die klinische Validierung an Liquorproben durchgeführt von sehr sorgfältig ausgewählten und charakterisierten Patienten mit Alzheimer Erkrankung (AD, Alzheimer disease) oder milden Wahrnehmungsstörungen (MCI, mild cognitive impairment) mit Alzheimer-Pathologie (MCl-AD) - hier Positivgruppe - und nicht dementen Kontrollen - hier Negativgruppe. AD/MCI-Patieneten wurden diagnostiziert und sub-klassifiziert entsprechend der aktuellsten Empfehlungen vom Nationalinstitut für Alterung (National Institute on Aging (NIA) - dabei der Alzheimer's Association (AA) working groups, Albert et al. [21], McKhann et al. [22]).

Noch ein Aspekt außerhalb der vorliegenden Erfindung bezieht sich auf die Verwendung der Sequenz TPP oder deren phosphorylierter Form T(Pi)PP zur Immunisierung von Säugern zur Erzeugung von Antikörpern gegen die Sequenz TPP. Diese Sequenz liegt vorzugsweise in Form von länger als diese Sequenz bestehenden Peptiden oder Proteinen oder auch einzeln als Sequenz ergänzt durch z. B. weitere Aminosäuren vor. Als Säuger sind Mäuse oder andere geeignete Tiere (insbesondere Ratten und Kaninchen) bevorzugt, um Antikörper oder andere Moleküle gegen Antigene, die die Sequenz TPP als Bindungstelle dieser Antikörper enthalten, gegen die Sequenz TPP zu bilden.

Ferner wird in einem Aspekt außerhalb der vorliegenden Erfindung auf die Verwendung der Sequenz TPP oder deren phosphorylierter Form T(Pi)PP zur Herstellung eines Therapeutikums oder zur Immunisierung beim Menschen Bezug genommen.

Schließlich wird in einem anderen Aspekt außerhalb der vorliegenden Erfindung die Verwendung der Sequenz TPP oder deren phosphorylierter Form T(Pi)PP zur Herstellung von chemischen Bindern beschrieben, die die Struktur des erfindungsgemäßen Epitopes TPP nachahmen.

Die vorstehenden Ausführungen und Bevorzugungen im Hinblick auf einen Aspekt der vorliegenden Erfindung gelten für die anderen dargestellten Aspekte entsprechend.

Weitere Ziele, Merkmale, Vorteile und Anwendungsmöglichkeiten ergeben sich aus der nachfolgenden Beschreibung von die Erfindung nicht einschränkenden Ausführungsbeispielen, auch anhand der Figuren. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbeziehung. Es zeigen:
- Fig. 1: eine schematische Darstellung von TAU-Isoformen,
- Fig. 2: eine Aggregation amyloidogener Proteine und Ausbildung von Poren in der Zellmembran,
- Fig. 3: eine ROC-Kurvenanalyse des Phosphor-bezogenen TAU ELISA (pTAU rel) und
- Fig. 4: Ergebnisse der Analysen von Patientenproben.

Nachfolgend wird ein konkretes, die Erfindung aber nicht einschränkendes Beispiel beschrieben.

Zunächst wurden Mäuse in Freund'schem Adjuvans immunisiert mit:
- rekombinant hergestelltem und *in vitro* aggregiertem TAU-Protein,
- rekombinant hergestelltem TAU-Protein, das mittels Kinasen *in vitro* phosphoryliert und *in vitro* aggregiert wurde,
- synthetischen Peptiden CIPAKTPPAPKTPPS und CKKVAVVRTPPKSPSS, die den Aminosäuresequenzen 170-184 bzw. 223-238 des TAU441 entsprechen, sowie
- synthetischen Peptiden CIPAKTPPAPKTPPS und CKKVAVVRTPPKSPSS, die an den Positionen T181 und T231 phosphoryliert wurden.

Den Mäusen wurden die B-Lymphozyten entnommen und mit der Myelomzelllinie P3X63 Ag8.653 unter Verwendung von 50 %igem Polyethylenglycol fusioniert.

Die Fusionsprodukte wurden mittels HAT-Selektion nach Littlefield [23] in Zellkulturplatten selektioniert. Die Charakterisierung der Primärzelllinie erfolgte durch folgende Testungen:
- Testung auf IgG-produzierende und wachstumfähige Hybridome,
- Prüfung der Reaktivität des produzierten IgG mit den Immunogenen im ELISA (Enzyme Linked Immunosorbent Assay) und Western Blot (auch "Immunblot") und
- Prüfung der Reaktivität des produzierten IgG mit dem TAU-Protein im ELISA und Western Blot.

Nach der Immunisierung und Charakterisierung wurden rekombinant hergestelltes TAU-Protein mittels Kinasen *in vitro* phosphoryliert und *in vitro* aggregiert und die synthetischen Peptide CIPAKTPPAPKTPPS und CKKVAVVRTPPKSPSS an den Positionen T181 und T231 phosphoryliert, um geeignete Antikörper zu erzeugen, die an eine Aminsoäuresequenz um die Phosphorylierungsstellen T181 bzw. T231 binden und zwar ausschließlich, wenn diese nicht phosphoryliert vorlagen.

Von den erzeugten Hybridomen wurden diejenigen ausgewählt, die darüber hinaus die besten Reaktionen mit dem rekombinanten TAU-Protein im ELISA und Western Blot zeigten und Antikörper vom angestrebten Isotyp IgG produzierten.

Die entwickelten Hybridome wurden nach Immunisierung, Fusion mit der Myelomzelllinie P3X63 Ag8.653 und HAT-Selektion kloniert und subkloniert und zu monoklonalen Hybridomzellen kultiviert. Expandierte monoklonale Hybridome wurden wiederum auf ihre Fähigkeit, IgG zu produzieren, und ihre Reaktivität gegenüber dem TAU-Protein mittels ELISA und Western Blot getestet. Nach erfolgreicher Testung wurden die Hybridome durch Einfrieren von 5 x 10⁶ Zellen in fetalem Kälberserum + 10 % Dimethylsulfoxid gesichert.

Aus der Vielzahl der entstandenen Hybridome wurde die erfindungsgemäße Zelllinie H-1G2 als produktiver Klon ausgewählt und kultiviert.

### Produktion und Sicherung des MAk und der erfindungsgemäßen Hybridomzelllinie H 1G2

Zunächst wurde eine Zellbank der erfindungsgemäßen monoklonalen Hybridomzelllinie H-1G2 angelegt. Dann wurden Chargen des erfindungsgemäßen monoklonalen Antikörpers 1G2 produziert und nach Prüfung zur Verwendung freigegeben.

### Charakterisierung des erfindungsgemäßen monoklonalen Antikörpers 1G2

Der erfindungsgemäße Antikörper 1G2 der erfindungsgemäßen monoklonalen Hybridomzelllinie H-1G2 wurde charakterisiert unter Verwendung eines Maus-Hybridoma-Subtyping Kit der Firma Roche. Das Ergebnis dieser Analyse zeigt, dass der Antikörper den Isotyp IgG2a[kappa] besitzt.

Westernblot-Analysen wurden durchgeführt und zeigten, dass der erfindungsgemäße Antikörper 1G2 mit TAU441 reagiert.

Epitopmapping erfolgte mittels Pepscan nach Osman et al. [24]. Der erfindungsgemäße Antikörper 1G2 erkannte das Epitop TPP mit direkt vor- oder nachgelagerter Aminosäure K an humanem TAU-Protein.

Reaktivitätsnachweise in Immuno-Assays mit nicht-phosphoryliertem TAU-Protein werden nachstehend in den Ausführungsbeispielen erbracht.

### Ausführunqsbeispiele

### 1) Phosphor-bezogener TAU ELISA

Der ELISA ist ein enzymimmunologischer Assay, der aus einem Fang-Antikörper (monoklonal), einer Präparation an TAU441 und einem POD-markierten Nachweis-Antikörper (monoklonal) besteht. Beide Antikörper binden an unterschiedlichen Bindungsdomänen des TAU441, so dass das TAU441 eine spezifische Brücke zwischen beiden Antikörpern (sandwich) bildet.

### Beschreibung der Durchführung des ELISA

Hundert Mikroliter des erfindungsgemäßen monoklonalen Antikörpers 1G2 wurden in 0,05 M Karbonatpuffer pH 9,6 in die Vertiefung einer Mikrotiterplatte (MTP) pipettiert und für 18 Stunden beladen. Der Inhalt wurde abgesaugt und die freien Bindungsstellen wurden mittels 3 %iger Rinderserumalbuminlösung (Assaypuffer) abgesättigt. 100 µl TAU441-Präparation in einer Verdünnungsreihe in Assaypuffer sowie 100 Liquorproben (58 Alzheimer-Patienten, 42 Kontroll-Patienten) in Doppelbestimmung, verdünnt 1:1 in Probenpuffer wurden in der Vertiefung der MTP gegeben und für 120 Minuten bei Raumtemperatur und unter schütteln (300 rpm) inkubiert. Nach Absaugen wurden 100 µl Nachweis-Antikörper-POD (Klon 7E5, spezifisch für TAU441, Eigentümer AJ Roboscreen GmbH) pipettiert und 90 Minuten bei Raumtemperatur inkubiert. Über eine nachgeschaltete Substrat-POD (Horseradishperoxidase)-Reaktion kann die gebundene Menge des TAU441 bestimmt werden. Als Substrat wird Tetramethylbenzidin (TMB) verwendet. Die Höhe des Signals ist proportional der Menge des vorhandenen TAU441. Die unbekannten Proben werden anhand ihrer Signalhöhe aus einer Eichkurve bestimmt.

### Ergebnis des ELISA

Es wurde im ELISA die Bindung von TAU441 an eine mit dem erfindungsgemäßen monoklonalen Antikörper 1G2 beschichtete Mikrotiterplatte gezeigt. Es wurde eine Kalibrierkurve anhand der Verdünnungsreihe an TAU441 aufgestellt mit deren Hilfe die gemessenen Liquorproben quantifiziert werden konnten. Es wurde gezeigt, dass die berechneten Konzentrationen an Phosphor-bezogenem TAU der Liquorproben sich signifikant zwischen den Gruppen der Alzheimer-Patienten, inkl. Patienten im präklinischen Stadium (109,2 ± 32 pg/ml) und den Kontroll-Patienten (62,1 ± 9,1 pg/ml) unterschieden p < 0.001 (Mann-Whitney Test).

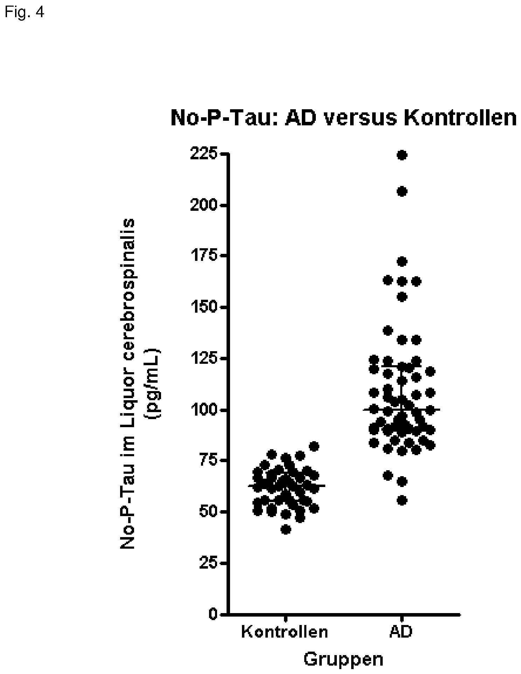

Figur 4 bildet die individuellen Konzentrationen (jeder Punkt stellt den Mittelwert der Doppelbestimmungen dar) und die Medianwerte mit Standardabweichungen (horizontale Balken) ab (vgl. auch Tabelle 2).

Die untersuchten Patientengruppen wiesen die in Tabelle 1 dargestellte Charakteristik auf.

**Tabelle 1**

| Gruppe | Männer/Frauen | Alter (Jahre) | MMSE |
|---|---|---|---|
| AD/MCI (n=58) mit | 23/35 | 70,1 (±8.2) | 23,1 |
| - wahrscheinliche AD mit einem hohen Nachweis an AD-pathophysiologischen Prozessen (n=24). | | | (±4,1) |
| - mögliche AD mit einem hohen Nachweis an AD-pathophysiologischen Prozessen (n=8). | | | |
| - MCI mit hoher Wahrscheinlichkeit eines AD Prozessen (n=26). | | | |
| Kontrollen (n=42) | 18/24 | 50,3 (±14.5) | NA |

| | | | |
|---|---|---|---|
| MMSE: Mini-Mental-Status-Test ("Mini-Mental-State-Examination") nach Folstein [25] AD: Alzheimer'sche Erkrankung ("Alzheimer Disease") MCI: leichte kognitive Beeinträchtigung ("Mild Cognitive Impairment") NA: Not applicable ("Nicht zutreffend"-?) | | | |

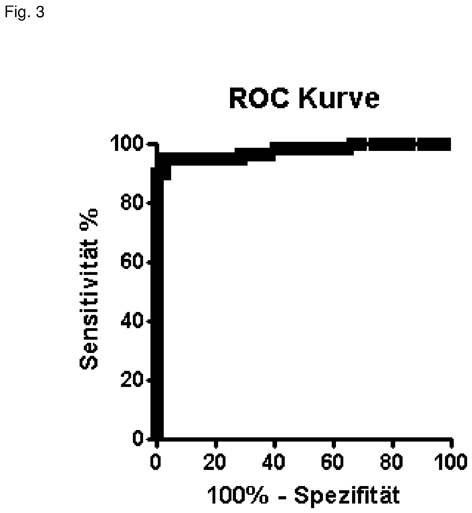

Bei der ROC-Kurven-Analyse und der Bestimmung der diagnostischen Spezifität und Sensitivität konnte gezeigt werden, dass der ELISA einen sehr hohen Youden-Index mit 0,9245 (Spanne zwischen 0 und 1) bei einer Spezifität von 97,6 % und einer Sensitivität von 94,8 % aufweist und die Fläche unter der ROC-Kurve 0,976 betrug, wie in Figur 3 gezeigt wird. Ein diagnostisch relevanter Grenzwert wurde zwischen 60 pg/ml und 90 pg/ml gefunden.

**Tabelle 2: Beschreibende Statistik der Ergebnisse der ELISA-Messungen in den Gruppen.**

| Variable | Gruppe | N | Mean | Standard-abweichung | Medianwert | Untere Quartile | Obere Quartile |
|---|---|---|---|---|---|---|---|
| (No-P-T)-AJ Roboscreen | Positivgruppe | 58 | 109,2 | 32,0 | 100,0 | 90,3 | 120,3 |
| (No-P-T)-AJ Roboscreen | Kontrollgruppe | 42 | 62,1 | 9,3 | 62,6 | 55,3 | 68,6 |

Anwendungsgebiete der Erfindung sind die Diagnostik und die Therapie von neurodegenrativen Erkrankungen und speziell deren häufigster Form, des *morbus Alzheimer.* Die Diagnostik kann in verschiedenen Körperflüssigkeiten oder Gewebsproben mittels immunochemischer Testverfahren erfolgen. Weiterhin kann der erfindunsgemäße monoklonale Antikörper 1G2 für histologische Untersuchungen *post mortem* in der neuropathologischen Diagnostik eingesetzt werden.

### Literaturquellen

[1] Köhler, G.; Milstein, C.; Continuous Cultures Of Fused Cells Secreting Antibody Of Predefined Specificity; Nature 256 (5517); 495-497; 1975
[2] Lewczuk, P., Kornhuber, J.; Neurochemical dementia diagnostics in Alzheimer's disease: where are we now and where are we going?; Expert Rev Proteomics; 2011 Aug; 8(4); 447-58
[3] Kosik, K. S., Crandall, J. E., Mufson, E. J., Neve, R. L.; Tau in situ hybridization in normal and Alzheimer brain: localization in the somatodendritic compartment; Ann. Neurol. 26; 352-361, 1989
[4] Goedert, M., Spillantini, M. G., Jakes, R., Rutherford D., Crowther ,R. A.; Multiple isoforms of human microtubule-associated prorein tau: sequences and localisation in neurofibrillary tangles of Alzheimer's disease; Neuron 3 ;519-526; 1989
[5] Neve, R. L., Harris, P., Kosik K. S., Kurnit, D. M., Donlon, A.; Identification of cDNA clones for the human microtubule- associated protein tau and chromosomal localisation of the genes for tau and microtubule-associated protein 2; Molecular Brain Research 1; 271-280; 1986
[6] Goedert, M., Crowther, R. A., Garner, C.C.; Molecular Characterization of Microtubule-Associated Proteins- Tau and Map2; Trends Neurosci 14; 193-199, 1991
[7] Goedert M.; Tau protein and the neurofibrillary pathology of Alzheimer's disease.; Trends Neurosci 16, 460-465; 1993
[8] Hutton, M., Lendon, C.L., Rizzu, P., Baker, M., Froelich, S., Houlden, H., Pickering-Brown, S., Chakraverty, S., Isaacs, A., Grover, A., Hackett, J., Adamson, J., Lincoln, S., Dickson, D., Davies, P., Petersen, R. C., Stevens, M., de Graaff, E., Wauters, E., van Baren, J., Hillebrand, M., Joosse, M., Kwon, J. M., Nowotny, P., Che, L. K., Norton, J., Morris, J. C., Reed, L. A., Trojanowski, J., Basun, H., Lannfelt, L., Neystat, M., Fahn, S., Dark, F., Tannenberg, T., Dodd, P. R., Hayward, N., Kwok, J. B., Schofield, P. R., Andreadis, A., Snowden, J., Craufurd, D., Neary, D., Owen, F., Oostra, B. A., Hardy, J., Goate, A., van Swieten, J., Mann, D., Lynch, T., Heutink, P.; Association of missense and 5'-splice-site mutations in tau with the inherited dementia FTDP-17. Nature 393; 702-705; 1998
[9] Bugiani, O., Murrell, J. R., Giaccone, R., Hasegawa, M., Ghigo, G., Tabaton, M., Morbin, M., Primavera, A., Carella, F., Solaro, C., Grisoli, M., Savoiardo, M., Spillantini, M. G., Tagliavini, F., Goedert, M., Ghetti, B.; Frontotemporal dementia and corticobasal degeneration in a family with a P301S mutation in tau; J Neuropathol Exp Neurol 58; 667-677; 1999
[10] Eidenmüller, J., Fath, T., Hellwig, A., Reed, J., Sontag, E., Brandt, R.; Structural and functional implications of tau hyperphosphorylation: information from phosphorylation-mimicking mutated tau proteins; Biochemistry 39; 13166-13175; 2000
[11] Bancher, C., Brunner, C., Lassmann, H., Budka, H., Jellinger, K., Wiche, G., Seitelberger, F., Grundke-Iqbal, I., Iqbal, K., Wisniewski, H. M.; Accumulation of abnormally phosphorylated tau precedes the formation of neurofibrillary tangles in alzheimer's disease; Brain Res. 477; 90-99; 1989
[12] Friedhoff, P., von Bergen, M., Mandelkow, E. M., Davies, P., Mandelkow, E.; A nucleated assembly mechanism of Alzheimer paired helical filaments; Proc.Natl.Acad.Sci.U.S.A 95; 15712-15717; 1998
[13] Chirita, C. H., Kuret, J.; Evidence for an intermediate in tau filament formation. Biochemistry 43; 1704-1714; 2004
[14] Berriman, J., Serpell, L. C., Oberg, K. A., Fink, A. L., Goedert, M., Crowther, R.A.: Tau filaments from human brain and from in vitro assembly of recombinant protein show cross-beta structure; Proc.Natl.Acad.Sci.U.S.A 100; 9034-9038; 2003
[15] Lashuel, H.A.; Membrane permeabilization: a common mechanism in proteinmisfolding diseases; Sci Aging Knowledge Environ 38; pe28; 2005
[16] Maeda, S., Sahara, N., Saito, Y., Murayama, M., Yoshiike, Y., Kim, H., Miyasaka, T., Murayama, S., Ikai, A., Takashima, A.; Granular tau oligomers as intermediates of tau filaments; Biochemistry 46; 3856-3861; 2007
[17] Caughey, B., Lansbury, P.T.; Protofibrils, pores, fibrils, and neurodegeneration: separating the responsible protein aggregates from the innocent bystanders; Annu. Rev. Neurosci. 26; 267-298; 2003
[18] Blennow, K., Dubois, B., Fagan, A. M., Lewczuk, P., de Leon, M. J., Hampel, H.; Clinical utility of cerebrospinal fluid biomarkers in the diagnosis of early Alzheimer's disease; Alzheimers Dement. 2; 5552-5260; 2014 May
[19] Braithwaite, S. P., Stock, J. B., Lombroso, P. J., Nairn, A. C.; Protein phosphatases and Alzheimer's disease; Prog Mol Biol Transl Sci; 106; 343-379; 2012
[20] Cambridge University Press; Recombinant Antibodies for Immunotherapy. Edited by Melvyn Little. July 2009
[21] Albert, M. S., DeKosky, S. T., Dickson, D., Dubois, B., Feldman, H. H., Fox, N. C., Gamst, A., Holtzman, D.M., Jagust, W. J., Petersen, R. C., Snyder, P. J., Carrillo, M. C., Thies, B., Phelps, C. H.; The diagnosis of mild cognitive impairment due to Alzheimer's disease: recommendations from the National Institute on Aging-Alzheimer's Association workgroups on diagnostic guidelines for Alzheimer's disease.; Alzheimers Dement. 7(3); 270-279; 2011
[22] McKhann, G. M., Knopman, D. S., Chertkow, H., Hyman, B. T., Jack, C. R. Jr., Kawas, C. H., Klunk, W. E., Koroshetz, W. J., Manly, J. J., Mayeux, R., Mohs, R. C., Morris, J. C., Rossor, M. N., Scheltens, P., Carrillo, M. C., Thies, B., Weintraub, S., Phelps, C. H.; The diagnosis of dementia due to Alzheimer's disease: recommendations from the National Institute on Aging-Alzheimer's Association workgroups on diagnostic guidelines for Alzheimer's disease.; Alzheimers Dement. 7(3); 263-269; 2011
[23] Littlefield, J. W.; Selection Of Hybride From Matings Of Fibroblasts In Vitro And Their Presumed Recombinants; Science 145; 709-710, 1964
[24] Osman, A. A., Uhlig, H. H., Valdes, I., Amin, M., Mendez, E., Mothes, T.; Monoclonal Antibody Recognizing A Potential Coeliac Toxic Repetitive Pentapeptide Epitope In Gliadins; Eur. J. Gastroenterol. Hepatol. 13; 1189-1193; 2001
[25] Folstein, M. F., Folstein, S. E., McHugh, P. R.; Mini-Mental State (a practical method for grading the state of patients for the clinician); Journal of Psychiatric Research 12; 189-198; 1975

## Patentansprüche

1. Monoklonaler Antikörper 1G2, produziert durch die Hybridomzelllinie H-1G2, **gekennzeichnet durch** DSM ACC3248.

2. Hybridomzelllinie H-1G2, **gekennzeichnet durch** DSM ACC3248.

3. Verwendung des monoklonalen Antikörpers 1G2 nach Anspruch 1 zur Erkennung in vitro einer Fraktion des TAU-Proteins, die an den Positionen T175 und T181 und T231 nicht phosphoryliert ist.

4. Verwendung nach Anspruch 3 zum Nachweis der Fraktion des TAU-Proteins in vitro in biologischen Proben von Menschen.

5. Immunochemisches Nachweissystem, umfassend
i) den monoklonalen Antikörper 1G2 nach Anspruch 1 als Fang-Antikörper,
ii) einen zweiten monoklonalen Antikörper gegen ein TAU-Protein als Nachweis-Antikörper,
iii) zumindest eine Kontrolle,
iv) zumindest eine Färbe- und/oder Nachweislösung,
v) zumindest eine Stopplösung,
vi) zumindest einen Probenpuffer,
vii) zumindest einen Waschpuffer und
viii) Mittel zur Probenvorbereitung.

6. Verwendung des immunochemischen Nachweissystems nach Anspruch 5 in der Humandiagnostik in vitro zur Untersuchung von Blutplasma, Blutserum, Liquor oder anderen Körperflüssigkeiten auf eine Fraktion des TAU-Proteins, die an den Positionen T175 und T181 und T231 nicht phosphoryliert ist.

7. Verwendung des immunochemischen Nachweissystems nach Anspruch 5 in der neurochemischen Demenzdiagnostik in vitro beim Menschen.

## Claims

1. A monoclonal antibody 1G2, produced by the hybridoma cell line H-1G2, **characterized by** DSM ACC3248.

2. A hybridoma cell line H-1G2 **characterized by** DSM ACC3248.

3. Use of the monoclonal antibody 1G2 according to claim 1 for recognizing in vitro a fraction of the TAU protein which is non-phosphorylated at positions T175 and T181 and T231.

4. The use according to claim 3 for detecting the fraction of the TAU protein in vitro in human biological samples.

5. An immunochemical detection system comprising
i) the monoclonal antibody 1 G2 according to claim 1 as capture antibody;
ii) a second monoclonal antibody against a TAU protein as detection antibody;
iii) at least one control;
iv) at least one staining and/or detection solution;
v) at least one stop solution;
vi) at least one sample buffer;
vii) at least one wash buffer; and
viii) means for sample preparation.

6. Use of the immunochemical detection system according to claim 5 in human diagnostics in vitro for examining blood plasma, blood serum, cerebrospinal fluid or other body fluids for a fraction of the TAU protein which is non-phosphorylated at positions T175 and T181 and T231.

7. Use of the immunochemical detection system according to claim 5 in the neurochemical dementia diagnostics in vitro in humans.

## Revendications

1. Anticorps monoclonal 1G2 produit par la lignée cellulaire d'hybridome H-1G2, **caractérisée par** le DSM ACC3248.

2. Lignée cellulaire hybride H-1G2, **caractérisée par** le DSM ACC3248.

3. Utilisation de l'anticorps monoclonal 1G2 selon la revendication 1 pour la détection in vitro d'une fraction de la protéine TAU qui n'est pas phosphorylée aux positions T175 et T181 et T231.

4. Utilisation selon la revendication 3 pour la détection de la fraction de la protéine TAU in vitro dans des échantillons biologiques provenant de l'homme.

5. Système de détection immunochimique comprenant
i) l'anticorps monoclonal 1G2 selon la revendication 1 comme anticorps de capture,
ii) un second anticorps monoclonal contre une protéine TAU comme anticorps de détection,
(iii) au moins un contrôle,
iv) au moins une solution de coloration et/ou de détection,
v) au moins une solution d'arrêt,
vi) au moins un tampon d'échantillonnage,
vii) au moins un tampon de lavage, et
(viii) des moyens pour la préparation des échantillons.

6. Utilisation du système de détection immunochimique selon la revendication 5 dans des diagnostics humains in vitro pour l'examen du plasma sanguin, du sérum sanguin, du liquide céphalorachidien ou d'autres fluides corporels pour une fraction de la protéine TAU liée aux
positions T175 et T181 et T231 qui n'est pas phosphorylée.

7. Utilisation du système de détection immunochimique selon la revendication 5 dans les diagnostics de démence neurochimique in vitro chez l'homme.
